# EUROPEAN PATENT SPECIFICATION

(11) **EP 2 124 611 B1**
(45) Date of publication and mention of the grant of the patent: **13.01.2016**
(21) Application number: 08730573.6
(22) Date of filing: 22.02.2008
(51) Int. Cl.: A23K 1/16, A23K 1/17, A23K 1/18

(54) **COMPOSITIONS FOR ALTERING GENE EXPRESSION IN CANINES**
ZUSAMMENSETZUNGEN ZUR ÄNDERUNG VON GENEXPRESSION IN HUNDEN
COMPOSITIONS POUR MODIFIER L'EXPRESSION GENIQUE CHEZ LES CANINS

(30) Priority: 22.02.2007 US 891171 P
(43) Date of publication of application: 02.12.2009
(73) Proprietor: Hill's Pet Nutrition, Inc., Topeka, KS 66605 (US)
(72) Inventor: YAMKA, Ryan Michael, Topeka, KS 66610 (US); FRIESEN, Kim Gene, Topeka, KS 66614 (US); ZICKER, Steven Curtis, Lawrence, KS 66049 (US); FRANTZ, Nolan Zebulon, Topeka, Kansas 66618 (US); GAO, Xiangming, Topeka, Kansas 66615 (US)
(74) Representative: Daniels, Jeffrey Nicholas
(86) International application number: PCT/US2008/054800
(87) International publication number: WO 2008/103958

(56) References cited:
- WO-A-2005/006877
- WO-A-2005/032271
- WO-A-2006/074089
- US-A1- 2002 001 640
- PEREZ-MATUTE P, PEREZ-ECHARRI N, MARTINEZ JA, MARTI A, MORENO-ALIAGA MJ: "Eicosapentaenoic acid actions on adiposity and insulin resistance in control and high-fat-fed rats: role of apoptosis, adiponectin and tumour necrosis factor alpha" BR. J. NUTR., vol. 97, 14 February 2007 (2007-02-14), pages 389-398, XP002487817
- MUTCH DM, GRIGOROV M, BERGER A, FAY LB, ROBERTS MA, WATKINS SM, WILLIAMSON G, GERMAN JB: "An integrative metabolism approach identifies stearoyl-CoA desaturase as a target for an arachidonate-enriched diet" FASEB J., vol. 19, no. 6, 24 January 2005 (2005-01-24), pages 599-601, XP002487818
- RINGSEIS R, EDER K: "Effects of dietary fat and oxidized cholesterol on gene expression in rat liver as assessed by cDNA expression array analysis" EUR. J. NUTR., vol. 44, 2005, pages 231-241, XP002487819

## Description

### FIELD OF THE INVENTION

The present invention relates to compositions and methods regulating gene expression or transcription in a growing animal.

### BACKGROUND OF THE INVENTION

Commercially available canine and feline foods include compositions specially formulated to address many different nutritional needs. These include, for example, formulations designed for different breed types, sizes and body conditions. They also include formulations designed to address the nutritional needs of animals in the different stages of their life cycle. Typically, these stages include the growth, adult and senior stages of life. For example, US Patent 5,851,573 discloses a pet food composition for large breed puppies; US Patent 6,426,100 discloses compositions to provide improved bone modeling and chondrocyte functioning in growing animals; US Patent 6,582,752 discloses gender specific puppy food. Despite the availability of such pet food formulations, however, the need remains for the development of additional formulations comprising innovative ingredients and nutrients designed to enhance the development of growing animals.

It is known in the art that certain nutrients have an effect on gene expression. Nutrigenomics is the study of such a relationship. Despite what is already known, there is a need to develop compositions and methods which may positively influence gene expression of an animal.

WO2005/032271 describes methods and kits for enhancing ability to learn in a puppy or kitten.

US2002/0001640 describes a method for improving bone modeling and chondrocyte functioning in growing canines.

### SUMMARY OF THE INVENTION

In certain aspects, the present description relates to compositions that are useful to enhance the development of a growing animal. Particularly, the compositions defined herein comprise one or more nutrients or bioactive substances that can enhance neurologic development, bone and joint health, immune function, and promote healthy body composition in a growing animal. In certain aspects, the nutrients and bioactive substances include, but are not limited to, fatty acids, antioxidants, essential nutrients, amino acids, minerals and trace elements, vitamins and vitamin-like substances. Other aspects of the description relate to methods to enhance the development of a growing animal comprising administration of effective amounts of the compositions defined herein directly to a growing animal or to the dam of said animal while the animal is *in utero* or is a nursling.

In one aspect, the present invention provides Composition 1.0, a pet food composition comprising:
about 5 to about 70% protein,
about 0.5 to about 1.6% methionine,
about 50 to about 200 ppm manganese,
about 0.1 to about 0.5% DHA,
about 0.1 to about 0.7% EPA,
about 1200 to about 7500 ppm choline,
about 1000 to about 2000 ppm taurine,
about 2.5 to about 6% linoleic acid,
about 1 to about 3% total n-3 fatty acids,
about 50 to about 1200 IU/kg vitamin E,
about 50 to about 500 ppm vitamin C,
about 50 to about 500 ppm carnitine, and
and about 2.5 to about 7 g lysine/1000 kcal,
wherein the composition is for use in preventing or treating arthritis or bone and joint inflammation, or in preventing or treating cancer in a canine,
wherein the use comprises regulating gene expression in the canine,
wherein the use comprises feeding a dam prior to and during pregnancy,
wherein the canine is born of the dam fed the composition prior to and during pregnancy,
and wherein the use further comprises feeding the canine the composition for at least one year post-partum.

The present invention also includes the following embodiments:
1.1 Composition for use as defined in 1.0 comprising:
   0 to about 90% by weight of carbohydrates;
   about 20% to about 60% by weight of protein;
   about 2% to about 50% by weight of fat;
   about 0.1% to about 20%, by weight of total dietary fiber; and
   0 to about 15% by weight of vitamins, minerals, and other nutrients in varying percentages which support the nutritional needs of the animal.
1.2 Composition for use as defined in 1.0 or 1.1 comprising about 5% to about 55%, by weight of carbohydrates;
1.3 Any of the preceding compositions for use as defined herein comprising about 20% to about 50%, by weight of protein, e.g., about 22% to about 50%;
1.4 Any of the preceding compositions for use as defined herein comprising about 5% to about 40%, by weight of fat, e.g., e.g., at least about 8% or about 9% to about 40% fat;
1.5 Any of the preceding compositions for use as defined herein comprising about 1% to about 11%, by weight of total dietary fiber;
1.6 Any of the preceding compositions for use as defined herein comprising about 0.1% to about 0.4% DHA, e.g., about 0.5%;
1.7 Any of the preceding compositions for use as defined herein comprising about 200 IU/kg to about 1200 IU/kg Vitamin E, e.g., about 200 IU/kg to about 1000 IU/kg;
1.9 Any of the preceding compositions for use as defined herein comprising about 100 ppm to about 500 ppm carnitine, e.g., about 200 to about 400, or about 300 ppm;
1.10 Any of the preceding compositions for use as defined herein comprising about 2.5 g/1000 kcal to about 7 g/1000 kcal lysine;
1.11 Any of the preceding compositions for use as defined herein comprising about 2500 ppm to about 7500 ppm choline, e.g., about 3000, about 3500, about 4000, about 4500, about 4600, about 4625, about 4650, about 4700, about 4800, or about 6000 ppm;
1.12 Any of the preceding compositions for use as defined herein comprising about 0.1 % to about 0.6 % EPA;
1.13 Any of the preceding compositions for use as defined herein comprising about 50 ppm to about 150 ppm manganese; and
1.14 Any of the preceding compositions for use as defined herein comprising about 0.8% to about 1.6% methionine.
1.15 In a preferred embodiment of the present invention, the use defined herein comprises feeding the dam the composition for a majority of the pregnancy duration.

The description further relates to methods to cause a beneficial modification in gene expression in an animal, specifically, down regulation in expression of a gene or genes associated with an undesirable biological condition or pathway or disease state and/or up regulation in expression of a gene or genes associated with a desired biological condition or pathway or which may have a positive or preventive effect on a disease state for any one or more biological conditions, pathways or disease states and genes described in Tables 2 - 15, comprising administering an effective amount of a composition of according to any one of compositions 1.0 -1.14 to an animal, either directly to the animal or to the dam while the animal is *in utero*.

In another aspect, the description provides Method 2.0, a method to regulate gene expression in a canine comprising administering to the canine any one of compositions the compositions of the present invention, e.g., compositions 1.0-1.14.

The present description also includes the following methods:
2.1 Of method 2.0 wherein the canine is a puppy.
2.2 Of method 2.0 or 2.1 wherein the canine is born of a dam fed any one of compositions 1.0 - 1.14 during pregnancy.
2.3 Of method 2.2 wherein the puppy is *in utero*.
2.4 Of method 2.2 wherein the dam is fed any one of compositions 1.0 - 1.14 prior to pregnancy.
2.5 Of method 2.2 or 2.4 wherein the dam is fed any one of compositions 1.0 -1.14 for a majority of the pregnancy duration.
2.6 Of any one of methods 2.2 - 2.5 wherein the dam is fed compositions consisting essentially of any one of compositions 1.0 -1.14 prior to and during pregnancy.
2.7 Of any one of the preceding methods wherein the puppy is fed any one of compositions 1.0 - 1.14 prior to weaning, e.g., while still a nursling.
2.8 Of any one of the preceding methods wherein the puppy is fed any one of compositions 1.0 - 1.14 post weaning.
2.9 Of method 2.8 wherein the puppy is fed food compositions consisting of any one of compositions 1.0 - 1.14.
2.10 Of any one of the preceding methods wherein an effective amount of the composition is administered to the canine.
2.11 Of any one of the preceding methods wherein the composition is administered to the canine for an effective amount of time.
2.12 Of method 2.11 wherein the composition is administered to the canine for at least one year post partum or one year post weaning.
2.13 Of any one of the preceding method wherein the gene is selected from those listed in Tables 2 - 15.

In a further aspect, the description relates to the use of any of the formulae of the present invention in the manufacture of a composition to modify gene expression in an animal as described herein.

Other features and advantages of the present invention will be understood by reference to the detailed description of the examples that follow.

### DETAILED DESCRIPTION OF THE INVENTION

Unless defined otherwise, all technical and scientific terms used herein have the same meanings as commonly understood by one of ordinary skill in the art to which this invention belongs. In addition, all references cited herein are hereby incorporated by reference in their entireties. In the event of a conflict in a definition in the present disclosure and that of a cited reference, the present disclosure controls.

As used herein and in the appended claims, the singular forms "a", "an", and "the" include plural reference unless the context clearly dictates otherwise.

The present description relates to any animal, preferably a mammal, more preferably a companion animal. The term "companion animal" refers to any animal that lives in close association with humans and includes, but is not limited to, canines and felines of any breed. It is contemplated herein, however, that any animal whose diet may be controlled by humans may benefit from feeding the formulations disclosed herein. These animals may include, for example, domesticated farm animals (e.g., cattle, horses, swine, etc.) as well as undomesticated animals held in captivity, e.g., in zoological parks and the like. Preferred animals include canines, e.g., dogs, including growing dogs, e.g., puppies. The present invention relates specifically to canines.

"Beneficial modification in the expression of genes" as used herein includes, e.g., down regulation of genes expressing proteins associated with disease states and/or up regulation of genes expressing proteins which have a beneficial or healthful effect as compared to appropriate controls, as may be determined using conventional methods, e.g., by microarray (e.g., Affymetrix gene chip) techniques familiar to one of skill in the art. Further, one of skill in the art is familiar with the known associations between diseases and specific genes as those listed in the tables provided hereinbelow such that it is understood whether an increase or decrease in expression of a particular gene is desirable.

The "growth" life stage of an animal refers to the period from birth or weaning (approximately 8 weeks of age) to about 1 year of age or beyond, depending on the species and breed of the animal.

As used herein, the term "puppy" refers to an immature canine, typically between the ages of birth and 12 months.

"Essential amino acids" as used herein refers to those amino acids that cannot be synthesized *de novo* by an organism and thus must be supplied in the diet. It is understood by one of skill in the art that the essential amino acids varies from species to species, depending upon the organism's metabolism. For example, it is generally understood that the essential amino acids for dogs and cats (and humans), are phenylalanine, leucine, methionine, lysine, isoleucine, valine, threonine, tryptophan, histidine and arginine.

As understood by one of skill in the art, a "limiting amino acid" refers to an amino acid which if present in insufficient quantities in a diet, results in the limitation in usefulness of other essential amino acids, even if the other essential amino acids are present in otherwise large enough quantities. Lysine is the limiting essential amino acid in the compositions disclosed herein. Thus, the remaining essential amino acids are quantitatively formulated or "balanced" in relationship to the amount of lysine determined critical to affect the desired biological result. As used herein, "balanced amino acids" refers to the relationship of the essential amino acid lysine to energy to assure optimal animal growth and development.

"Essential nutrients" as used herein refers to nutrients required for normal body functioning that cannot be synthesized by the body. Categories of essential nutrient include vitamin dietary minerals, fatty acid, and amino acid. It is understood by one of skill in the art that the nutrients deemed essential varies from species to species, depending upon the organism's metabolism. For example, essential nutrients for dogs and cats include Vitamins A, D, E, K, B1, B6 B12, riboflavin, niacin, pantothenic acid, folic acid, calcium, phosphorous, magnesium, sodium, potassium, chlorine, iron, copper, zinc, manganese, selenium and iodine. Choline, generally regarded as a B complex vitamin, may be included among the semi-essential nutrients. In addition, taurine, while technically not an amino acid but a derivative of cysteine, is an essential nutrient for cats.

Carnitine, also known as L-carnitine, (levocarnitine) is a quaternary ammonium compound synthesized from the amino acids lysine and methionine and is responsible for the transport of fatty acids from the cytosol into the mitochondria.

Without being limited to any theories or particular modes of action, the present invention is based on the surprising discovery that the addition of certain ingredients to pet food compositions and administration of these compositions to animals can enhance the development of a growing animal. For example, data indicate that animals fed the compositions as defined herein (or those whose dams were fed the compositions during gestation and prior to weaning but continued throughout growth of their litters), demonstrate enhanced neurologic development, bone and joint health, immune function, and have overall healthier body composition. Interestingly, microarray data also indicate a differential change in gene expression in these animals compared to controls which is generally reflective of a beneficial modification (i.e., up or down regulation) in the expression of many genes associated with biological processes including those involved in growth and development. Thus, in one aspect, the invention relates to compositions for use in preventing or treating bone arthritis or bone and joint inflammation, or in preventing or treating cancer in a canine.

As contemplated herein, in some embodiments, the compositions for use according to the present invention comprise nutritionally complete and balanced animal feed compositions. Such compositions include, among other nutrients and ingredients, recommended healthful amounts of protein, carbohydrate and fat. "Nutritionally complete and balanced animal feed compositions", as well as nutrients and ingredients suitable for animal feed compositions, and recommended amounts thereof, are familiar to one of skill in the art (see, for example, National Research Council, 2006 Nutritional Requirements for Dogs and Cats, National Academy Press, Washington D.C. or the Official Publication of the Association of American Feed Control Officials, Inc. Nutrient Requirements for Dogs and Cats 2006.

It is contemplated herein that the compositions disclosed herein may also comprise antioxidants, additives, stabilizers, thickeners, flavorants, palatability enhancers and colorants in amounts and combinations familiar to one of skill in the art. "Antioxidants" refers to a substance that is capable of reacting with or decreasing the production of free radicals and neutralizing them. Examples include, but are not limited to, beta-carotene, selenium, coenzyme Q10 (ubiquinone), lutein, tocotrienols, soy isoflavones, S-adenosylmethionine, glutathione, taurine, N-acetylcysteine, vitamin E, vitamin D, vitamin C, flavanoids, anthocyanindins, and lipoic acid.

While foods of any consistency or moisture content are contemplated, preferably the compositions of the present invention may be, for example, a wet, semi-dry, or dry animal food composition. "Wet" food refers to food which is sold in cans or foil bags and has a moisture content of about 70 to about a 90%. "Dry" food refers to compositions with about 5 to about 15% moisture content and is often manufactured in the form of small bits or kibbles. Semi-dry compositions refers to food which has a moisture content greater than dry foods, but less than wet foods. Also contemplated herein are compositions of intermediate moisture consistency and those that may comprise components of various consistency as well as components that may include more than one consistency, for example, soft, chewy meat-like particles as well as kibble having an outer cereal component and an inner cream component as described in, e.g., US Patent 6,517,877.

Various processes for manufacturing and packaging the compositions for use according to the present invention may be employed and are familiar to one of skill in the art.

It is also contemplated herein that the methods of the present description include methods to cause a beneficial modification in gene expression in an animal, specifically, down regulation in expression of a gene or genes associated with an undesirable biological condition or pathway or disease state and/or up regulation in expression of a gene or genes associated with a desired biological condition or pathway or which may have a positive or preventive effect on a disease state, as the case may be, for any one or more biological conditions, pathways or disease states and genes described in Tables 14-27, comprising administering an effective amount of a composition of the present invention to the animal, either directly to the animal or to the dam while the animal is *in utero.* Indeed, as discussed in Examples 7 and 8 here-in-below, by effecting the animal at the genomic level, the administration of the compositions of the present invention, either directly to the animal or to the dam while the animal is *in utero,* may have beneficial, even prophylatic, health effects on the animal by effecting the expression of any one or more genes listed in Tables 14-27. For example, the administration of an effective amount of a composition as defined herein to an animal may enhance bone and joint health in the animal by causing the down regulation in genes associated with cartilage and joint damage associated with arthritis, e.g. interleukin 1-beta, fibronectin, lactoransferrin, etc. (see Table 14).

It is contemplated herein that the compositions for use according to the present invention may be administered to an animal alone as a complete nutritionally balanced diet, or in conjunction with dietary supplements, vitamins and/or other nutritionally beneficial agents familiar to one of skill in the art, as part of an overall wellness program for the animal. Compositions for use according to the invention may also be useful as a veterinary therapeutic product. As such, the compositions may optionally contain a carrier, diluent, or an excipient, the suitability of which for the intended use being familiar to one of skill in the art.

In one aspect of the description, in addition to administering the compositions disclosed herein directly to a growing animal, e.g., to a growing puppy or kitten, the compositions may be administered to the dam of the animal while the animal is still *in utero* or while the animal is a nursling.

The following examples further illustrate the present invention and are not intended to limit the invention. As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

Except to the extent stated otherwise, all percentages used in this specification are weight percentages on a dry matter basis. The phrase "dry matter basis" means the component concentration in the composition after any moisture in the composition is removed.

### EXAMPLES

### Example 1

### Canine Genetic Effects: Conception to one year of age

In order to characterize the genotypic effects of feeding a composition formulated to enhance the development of a growing animal, an experiment is performed to identify changes in gene expression in puppies at one year of age from dams fed the compositions disclosed in Table 1.

**Table 1. Overage of analyzed nutrient profiles of foods utilized in the study**

| **Nutrients, 100% Dry Matter Basis** | **Composition H** | **Composition P** |
|---|---|---|
| Moisture, % | 6.09 | 7.44 |
| Crude Protein, % | 29.4 | 27.9 |
| Fat, % | 17.6 | 14.2 |
| Ca, % | 1.43 | 1.46 |
| P, % | 1.2 | 0.96 |
| EPA, % | 0.31 | <0.01 |
| DHA% | 0.19 | 0.01 |
| Linoleic Acid, % | 3.8 | 2.14 |
| Total n-3 fatty acids, % | 1.4 | 0.19 |
| Total n-6 fatty acids, % | 3.7 | 2.6 |
| Taurine, % | 0.14 | 0.08 |
| Carnitine, ppm | 312.1 | No analysis |
| Methionine, % | 1.3 | 0.52 |
| Cystine, % | 0.4 | 0.39 |
| Manganese, % | 94.5 | 64 |
| Vitamin E, IU/kg | 816 | 43.8 |
| Vitamin C, ppm | 168.6 | <10 |
| Choline, ppm | 4876 | No analysis |

Composition P is a commercially available dog food. Composition H is an experimental dog food composition.

Dams are fed either Composition H or Composition P for at least 10 days prior to conception. Dams are maintained in group lodging until confirmed pregnant via palpation, and are then moved to maternity lodging. Puppies from dams are kept on the same foods fed to the dams until one year of age (including pre-weaning and post-weaning period). Blood samples are then taken from the puppies and mRNA isolated according to conventional methods. Microarray assays are performed using the Affymetrix Canine- 2 gene chip according to conventional methods.

The tables below show the genes grouped by function and the direction of expression, wherein up regulation in Composition H ("H") vs Composition P ("P") demonstrates increased gene expression in Composition H fed puppies compared to Composition P fed puppies. Similarly, down regulation of a gene in the Composition H fed puppies vs Composition P fed puppies represents decreased gene expression in puppies fed Composition H relative to those fed Composition P.

**Table 2. Genes associated with arthritis/inflammation relevant to bone and joints**

| **Annotation** | **Probe** | **Direction of expression HvsP** |
|---|---|---|
| Interleukin 1-beta | CfaAffx.11741.1.S1_s_at | down |
| Fibronectin | Cfa.3707.1.A1_s_at | down |
| v-fos FBJ murine osteosarcoma viral oncogene homolog | Cfa.9039.1.A1_at | down |
| dual specificity phosphatase 1 | CfaAffx.25714.1.S1_s_at | down |
| Lactotransferrin | CfaAffx.21286.1.S1_s_at | down |
| compliment component 5a receptor | CfaAffx.7180.1.S1_s_at | down |
| xanthine dehydrogenase | CfaAffx.9452.1.S1_s_at | down |
| Secreted phoshoprotein 1 | CfaAffx.15042.1.S1_s_at | down |

**Table 3. Genes associated with DNA replication and repair**

| **Annotation** | **Probe** | **Direction of expression H vs P** |
|---|---|---|
| DNA fragmentation Interleukin 1-beta | CfaAffx.11741.1.S1_s_at | down |
| Fibronectin | Cfa.3707.1.A1_s_at | down |
| Neuroregulin 1 | CfaAffx.10523.1.S1_s_at | down |
| Dual specificity phosphatase 1 | CfaAffx.25714.1.S1_s_at | down |
| Cell death-inducing DFFA-like effector a | CfaAffx.28752.1.S1_at | down |
| **Proper DNA processing control** | | |
| Budding uninhibited by benzimidazoles 1 homolog | Cfa.1559.1.A1_at | up |
| Topoisomerase II alpha | Cfa.18946.1.S1_s_at | up |
| Kinetochore associated 2 | Cfa.3066.1.S1_s_at | up |
| Claspin homolog | CfaAffx.6235.1.S1_s_at | up |
| Cyclin B1 | CfaAffx.12419.1.S1_s_at | up |
| Cyclin B2 | Cfa.11939.1.A1_s_at | up |
| Human chromosome condensation protein G | CfaAffx.25509.1.S1_at | up |
| Leucine zipper protein 5 | CfaAffx.8781.1.S1_s_at | up |
| Kinesin family member 23 | Cfa.15293.1.A1_at | up |
| Flap structure specific endonuclease 1 | Cfa.1854.1.A1_at | up |

**Table 4. Genes associated with cancer**

| **Annotation** | **Probe** | **Direction of expression H vs P** |
|---|---|---|
| Interleukin 1-beta | CfaAffx.11741.1.S1_s_at | down |
| Fibronectin | Cfa.3707.1.A1_s_at | down |
| v-fos FBJ murine osteosarcoma viral oncogene homolog | Cfa.9039.1.A1_at | down |
| dual specificity phosphatase 1 | CfaAffx.25714.1.S1_s_at | down |
| compliment component 5a receptor | CfaAffx.7180.1.S1_s_at | down |
| xanthine dehydrogenase | CfaAffx.9452.1.S1_s_at | down |
| cellular repressor of E1A-stimulated genes 1 | Cfa.10558.3.A1_at | down |
| chondroitin sulfate proteoglycan 2 | CfaAffx.13597.1.S1_s_at | down |
| transcobalamin II, mycrocytic anemia | CfaAffx.19852.1.S1_s_at | down |
| serpin peptidase inhibitor, clade B member 10 | CfaAffx.1043.1.S1_s_at | down |
| adenomatosis polyposis coli down-regulated 1 | CfaAffx.28621.1.S1_at | down |
| six membrane epithelial antigen of the prostate 2 | Cfa.1933.1.S1_at | down |
| alanyl aminopeptidase | Cfa.3774.1.A1_s_at | down |
| Cancer susceptibility candidate 5 | Cfa.1126.1.S1_at | up |
| Paternally expressed 3 | Cfa.4482.1.S1_at | up |
| Topoisomerase II alpha | Cfa.18946.1.S1_s_at | up |
| Tubulin alpha 6 | CfaAffx.13667.1.S1_at | up |
| Cyclin B1 | CfaAffx.12419.1.S1_s_at | up |
| Cell division cycle associated 1 | CfaAffx.20529.1.S1_at | up |
| Cell division cycle 2 | CfaAffx.20006.1.S1_at | up |
| Ribonucleotide reductase M2 polypeptide | CfaAffx.6059.1.S1_at | up |
| Breast cancer anti-estrogen resistance 3 | Cfa.651.1.S1_at | up |
| Tumor necrosis factor receptor superfamily member 17 | CfaAffx.28790.1.S1_at | up |
| Protein kinase C, iota | CfaAffx.22822.1.S1_s_at | up |

**Table 5. Genes associated with cell compromise**

| **Annotation** | **Probe** | **Direction of expression H vs P** |
|---|---|---|
| Interleukin 1-beta | CfaAffx.11741.1.S1_s_at | down |
| Fibronectin | Cfa.3707.1.A1_s_at | down |
| Neuroregulin 1 | CfaAffx.10523.1.S1_s_at | down |
| v-fos FBJ murine osteosarcoma viral oncogene homolog | Cfa.9039.1.A1_at | down |
| dual specificity phosphatase 1 | CfaAffx.25714.1.51_s_at | down |
| compliment component 5a receptor | CfaAffx.7180.1.S1_s_at | down |
| xanthine dehydrogenase | CfaAffx.9452.1.S1_s_at | down |
| Macrophage receptor with collagenous structure | Cfa.15713.1.A1_s_at | down |
| Cyclin B1 | CfaAffx.12419.1.S1_s_at | up |
| Cell division cycle 2 | CfaAffx.20006.1.S1_at | up |
| Hemoglobin epsilon 1 | CfaAffx.10240.1.S1_s_at | up |
| Protein kinase C, iota | CfaAffx.22822.1.S1_s_at | up |

**Table 6. Genes associated with cellular assembly**

| **Annotation** | **Probe** | **Direction of expression H vs P** |
|---|---|---|
| Interleukin 1-beta | CfaAffx.11741.1.S1_s_at | down |
| Fibronectin | Cfa.3707.1.A1_s_at | down |
| Neuroregulin 1 | CfaAffx.10523.1.S1_s_at | down |
| chondroitin sulfate proteoglycan 2 | CfaAffx.13597.1.S1_s_at | down |
| cholinergic receptor, muscarinic 3 | Cfa.8414.1.A1_s_at | down |
| xanthine dehydrogenase | CfaAffx.9452.1.S1_s_at | down |
| Macrophage receptor with collagenous structure | Cfa.15713.1.A1_s_at | down |
| Cyclin B1 | CfaAffx.12419.1.S1_s_at | up |
| Cell division cycle 2 | CfaAffx.20006.1.S1_at | up |
| Leucine zipper protein 5 | CfaAffx.8781.1.S1_s_at | up |
| Protein kinase C, iota | CfaAffx.22822.1.S1_s_at | up |
| Kinesin family member 23 | Cfa.15293.1.A1_at | up |
| Budding uninhibited by benzimidazoles 1 homolog | Cfa.1559.1.A1_at | up |
| Cyclin B2 | Cfa.11939.1.A1_s_at | up |
| Topoisomerase II alpha | Cfa.18946.1.S1_s_at | up |
| Kinetochore associated 2 | Cfa.3066.1.S1_s_at | up |
| Cell division cycle associated 1 | CfaAffx.20529.1.S1_at | up |
| Human chromosome condensation protein G | CfaAffx.25509.1.S1_at | up |
| Kinesin family member 11 | CfaAffx.12118.1.S1_s_at | up |
| A kinase (PRKA) anchor protein 2 | Cfa.10574.1.A1_at | up |
| thymopioten | Cfa.18367.2.S1_s_at | up |

**Table 7. Genes associated with cell cycle regulation**

| **Annotation** | **Probe** | **Direction of expression H vs P** |
|---|---|---|
| Interleukin 1-beta | CfaAffx.11741.1.S1_s_at | down |
| Fibronectin | Cfa.3707.1.A1_s_at | down |
| Neuroregulin 1 | CfaAffx.10523.1.S1_s_at | down |
| v-fos FBJ murine osteosarcoma viral oncogene homolog | Cfa.9039.1.A1_at | down |
| cholinergic receptor, muscarinic 3 | Cfa.8414.1.A1_s_at | down |
| dual specificity phosphatase 1 | CfaAffx.25714.1.S1_s_at | down |
| cellular repressor of E1A-stimulated genes 1 | Cfa.10558.3.A1_at | down |
| Cyclin B1 | CfaAffx.12419.1.S1_s_at | up |
| Cell division cycle 2 | CfaAffx.20006.1.S1_at | up |
| Leucine zipper protein 5 | CfaAffx.8781.1.S1_s_at | up |
| centromere protein F, 350/400ka (mitosin) | CfaAffx.19534.1.S1_at | up |
| Breast cancer anti-estrogen resistance 3 | Cfa.651.1.S1_at | up |
| Budding uninhibited by benzimidazoles 1 homolog | Cfa.1559.1.A1_at | up |
| Cyclin B2 | Cfa.11939.1.A1_s_at | up |
| Topoisomerase II alpha | Cfa.18946.1.S1_s_at | up |
| Kinetochore associated 2 | Cfa.3066.1.S1_s_at | up |
| Cell division cycle associated 1 | CfaAffx.20529.1.S1_at | up |
| Human chromosome condensation protein G | CfaAffx.25509.1.S1_at | up |
| Kinesin family member 11 | CfaAffx.12118.1.S1_s_at | up |
| thymopioten | Cfa.18367.2.S1_s_at | up |

**Table 8. Genes associated with various functions**

| **Annotation** | **Probe** | **Direction of expression H vs P** |
|---|---|---|
| calcyphosine-like | Cfa.21214.1.S1_s_at | down |
| ELKS/RAB6 interacting/CAST family member 2 | Cfa.21214.1.S1_at | down |
| glutamyl tRNA synthetase 2 | Cfa.2057.1.A1_at | down |
| Coiled-coil containing domain 3 | Cfa.9084.1.A1_at | down |
| chromosome 16 clone RP11-26P10 | Cfa.3834.1.S1_at | down |
| SAM domain- and HD domain-containing protein 1 | CfaAffx.13689.1.S1_at | down |
| AJ420591 | Cfa.15196.1.A1_at | down |
| family with sequence similarity 46, member A | CfaAffx.5194.1.S1_at | down |
| CD1a antigen | Cfa.18390.1.S1_s_at | down |
| epidermal dendritic cell marker (CD1a) | CfaAffx.17796.1.S1_at | down |
| ANKYRIN MOTIF | CfaAffx.6104.1.S1_s_at | down |
| RP11-549H3 on chromosome X | Cfa.9597.1.S1_at | down |
| La ribonucleoprotein domain family, member 1 | Cfa.14608.1.A1_at | down |
| adenomatosis polyposis coli down-regulated 1 | CfaAffx.28621.1.S1_at | down |
| Chromosome 3 open reading frame 21 | CfaAffx.20763.1.S1-at | down |
| EGF-like module containing, mucin-like, hormone receptor-like 1 | CfaAffx.28381.1.S1_s_at | down |
| acyltransferase like 1 | CfaAffx.14833.1.S1_at | down |
| RP11-95J15 from 7 | Cfa.6138.1.A1_at | down |
| CD8 antigen, beta polypeptide 2 | Cfa.21011.1.S1_at | down |
| multimerin 1 | CfaAffx.15515.1.S1_s_at | down |
| none | Cfa.12500.1.A1_at | down |
| Hypothetical protein FLJ21062 | Cfa.1933.2.A1_at | down |
| normal mucosa of esophagus specific 1 | Cfa.11815.1.A1_at | down |
| regulator of G-protein signalling 7 | CfaAffx.24021.1.S1_s_at | down |
| Islet cell auto-antigen 1 normal mucosa of esophagus specific 1 (NMES1), | Cfa.11691.1.A1_at | down |
| transcript variant 2 | CfaAffx.851.1.S1_s_at | down |
| none | Cfa.5989.1.A1_s_at | down |
| eukaryotic translation initiation factor 2, subunit 3 gamma | Cfa.9060.1.A1_at | down |
| Serine/threonine kinase 17A | CfaAffx.21987.1.S1_at | down |
| RP11-542F9 on chromosome 6 | CfaAffx.21067.1.S1_at | up |
| chromosome 20 open reading frame 172 | CfaAffx.13625.1.S1_at | up |
| RP11-111I12 on chromosome 1 | CfaAffx.16431.1.S1_at | up |
| BAT2 domain containing 1 | Cfa.15064.1.S1_at | up |
| Rho GTPase activating protein 11A | CfaAffx.13079.1.S1_s_at | up |
| polo-like kinase 4 | CfaAffx.6762.1.S1_s_at | up |
| NADH dehydrogenase (ubiquinone) flavoprotein 3 | CfaAffx.16432.1.S1_s_at | up |
| 3 BAC RP11-223L18 | Cfa.3066.1.S1_at | up |
| par-3 partitioning defective 3 homolog B | Cfa.12402.1.S1_a_at | up |
| Hypothetical protein MGC24039 | Cfa.19506.1.S1_at | up |

| **Annotation** | **Probe** | **H vs P** |
|---|---|---|
| immunoglobulin mu heavy chain | CfaAffx.539.1.S1_x_at | up |
| origin recognition complex, subunit 1-like | Cfa.8552.1.A1_s_at | up |
| RP11-653G16 from 4 | CfaAffx.15773.1.S1_at | up |
| abnormal spindle-like microcephaly-associated protein | Cfa.103.1.A1_s_at | up |
| Solute carrier family 22 | Cfa.10558.2.S1_at | up |
| BCSynL32 immunoglobulin lambda light chain | CfaAffx.346.1.S1_at | up |
| Purinergic receptor P2Y, G-protein coupled 10 | Cfa.1521.1.S1_at | up |
| DEP domain containing 1 | CfaAffx.31279.1.S1_at | up |
| chromosome 17, clone RP11-160L11 | Cfa.16355.1.S1_at | up |
| Hypothetical protein LOC644115 | Cfa.15220.2.A1_at | up |
| isolate MRPS17P1 mitochondrial ribosomal protein S17 | Cfa.12402.1.S1_s_at | up |
| KIAA0101 (L5) | CfaAffx.26141.1.S1_s_at | up |
| chromosome 13 open reading frame 3 | CfaAffx.11656.1.S1_at | up |
| SHC SH2-domain binding protein 1 | CfaAffx.6499.1.S1_at | up |
| CDC20 cell division cycle 20 homolog | CfaAffx.8850.1.S1_s_at | up |
| Chromosome 14 open reading frame 32 | CfaAffx.23135.1.S1_s_at | up |
| 3-oxoacyl-ACP synthase, mitochondrial | Cfa.10049.1.A1_s_at | up |
| MAD2 mitotic arrest deficient-like 1 | CfaAffx.25205.1.S1_s_at | up |
| thioredoxin domain containing 5 | Cfa.10753.1.A1_at | up |
| Apla tubulin | Cfa.6991.1.A1_at | up |
| none | CfaAffx.32.1.S1_at | up |
| Melanin-concentrating hormone receptor 1 | Cfa.6032.1.A1_at | up |
| Aquaporin 7 | Cfa.21549.1.S1_s_at | up |
| cathepsin E | CfaAffx.15966.1.S1_at | up |
| EF hand domain (C-terminal) containing 2 | Cfa.1885.1.A1_at | up |
| bone morphogenetic protein 6 | Cfa.15702.1.S1_at | up |
| hemoglobin, alpha 2 | Cfa.3973.2.A1_s_at | up |
| SH3-domain GRB2-like 3 | Cfa.10644.1.A1_at | up |
| Acidic nuclear phosphoprotein 32 family, member A | Cfa.31.1.S1_s_at | up |
| none | CfaAffx.20953.1.S1_x_at | up |
| Rh family, B glycoprotein | Cfa.13613.1.A1_at | up |
| putative protein STRF8 | CfaAffx.15063.1.S1_s_at | up |
| fatty acid desaturase 1 | Cfa.13257.1.A1_at | up |
| Ligand dependent nuclear receptor corepressor-like | Cfa.1476.1.A1_at | up |
| F-box only protein 2 | CfaAffx.9335.1.S1_at | up |
| Ring finger protein 152 | Cfa.15414.1.A1_at | up |
| immunoglobulin lambda constant 1 | CfaAffx.22878.1.S1_at | up |
| G-protein coupled receptor 158 | Cfa.9162.1.A1_at | up |
| Hypothetical protein MGC42105 | CfaAffx.28387.1.S1_at | up |

Gene expression profiles from puppies fed Composition H and P are obtained and compared. Results indicate that 143 genes are differentially expressed between the study groups and, in general, their functions may be associated with cell assembly, cell cycle regulation, DNA replication and repair, cell compromise, arthritis and cancer. At a minimum of 1.5 fold change, 143 genes are differentially expressed in the two groups.

Genes associated with arthritis/inflammation are down-regulated in puppies fed Composition H compared to puppies fed Composition P, which suggests reduced cartilage/joint damage in Prototype pups. A separate study performed also indicates that levels of bone alkaline phosphatase are lower in puppies fed composition H (data not shown). See Table 2.

Genes associated with DNA fragmentation are down-regulated and genes associated with DNA processing control are up-regulated in puppies fed Composition H compared to puppies fed Composition P, which suggests reduced DNA damage and improved DNA protection in Prototype pups (also supported by elevated Vitamin E levels in blood, data not shown). See Table 3.

Genes associated with cancer incidence are down-regulated and genes associated with tumor suppression are up-regulated in puppies fed Composition H compared to puppies fed Composition P, which suggests the possibility of reduced cancer susceptibility in these Prototype animals; see Table 4.

Genes associated with cellular compromise are down-regulated and genes associated with cellular integrity are up-regulated in puppies fed Composition H compared to puppies fed Composition P, which suggests the possibility of reduced cell damage and enhanced cellular protection in these Prototype animals (also supported by elevated Vitamin E levels in blood, data not shown). See Table 5.

Genes associated with disruption of cellular assembly are down-regulated and genes associated with proper cellular organization are up-regulated in puppies fed Composition H compared to puppies fed Composition P, which suggests improved cellular organization and function in Prototype pups. See Table 6.

Genes associated with proper cell cycle regulation are up-regulated and genes associated with disruption of cell cycle control are down-regulated in puppies fed Composition H compared to puppies fed Composition P, which suggests proper control of cell cycle progression and cell survival. See Table 7.

Thus, it is contemplated herein that the nutritional benefits of the compositions of the present invention as described herein may involve modification in gene expression which results in the enhancement of the development of a growing animal. In addition, feeding proper nutrients during early development may have a prophylatic effect and influence disease processes later in life, i.e., lessen the chance of disease in the animal, as the expression of genes associated with common diseases and disorders may be influenced due to the given nutritional compositions of the foods provided maternally and during early development.

### Example 2

An experiment is performed to determine the effects on gene expression in puppies fed Composition H vs. Composition P from weaning until one year of age.

Dams utilized in this study are fed Composition P prior to, and during pregnancy. Following weaning, puppies are divided into two groups and provided with either Composition H or maintained on Composition P until one year old. Blood samples are then taken from the puppies and mRNA isolated according to conventional methods. Microarray assays are performed using the Affymetrix Canine- 2 gene chip according to conventional methods.

Results indicate that 99 genes are differentially expressed between the two study groups, and are presented in Tables 9 - 15. Of the genes identified, many are related to biological functions or pathways such as, e.g., immune activation, lipid metabolism, cardiovascular development, skeletal and muscular disorders, contraction and function and cell compromise and cancer.

**Table. 9 Genes associated with immune activation**

| | | **Direction of expression** |
|---|---|---|
| **Annotation** | **Probe** | **H v. P** |
| lymphocyte antigen 6 complex | | |
| | CfaAffx.1839.1.S1_at | down |
| Fc fragment of IgG, receptor, transporter, alpha | | |
| | Cfa.17806.2.S1_s_at | up |
| fibrinogen-like 2 | | |
| | Cfa.10303.1.S1_at | down |
| CD1A antigen | | |
| | Cfa.18390.1.S1_s_at | down |
| complement factor D (adipsin) | | |
| | Cfa.21381.1.S1_s_at | down |
| CD163 antigen; macrophage-associated antigen | | |
| | Cfa.9647.1.A1_at | down |
| toll-like receptor 2 | | |
| | CfaAffx.13248.1.S1_s_at | down |
| lysozyme (renal amyloidosis) | | |
| transcription elongation factor A (SII), 3 | CfaAffx.1598.1.S1_s_at | down |
| | Cfa.12580.1.51_s_at | down |
| fibronectin 1 | | |
| | Cfa.3707.2.S1_at | down |
| macrophage receptor with collagenous structure | | |
| | Cfa.15713.1.A1_s_at | down |
| bactericidal/permeability-increasing protein | | |
| | CfaAffx.14056.1.S1_s_at | down |
| T cell receptor alpha locus | | |
| | Cfa.10333.1.A1_at | down |
| immunoglobulin heavy constant alpha 2 | | |
| | Cfa.4556.3.A1_a_at | down |
| nuclear factor I/B | | |
| | Cfa.4487.1.S1_at | up |

**Table. 10 Genes associated with Cancer formation**

| | | **Direction of expression** |
|---|---|---|
| **Annotation** | **Probe** | **P vs C** |
| fibronectin 1 | Cfa.3707.3.S1_s_at | down |
| xanthine dehydrogenase | CfaAffx.9452.1.S1_s_at | down |
| neuregulin 1 | CfaAffx.10523.1.S1_s_at | down |
| colony stimulating factor 1 receptor | CfaAffx.27899.1.S1_at | down |
| alanyl aminopeptidase | Cfa.3774.1.A1_s_at | down |
| S100 calcium binding protein | CfaAffx.22128.1.S1_at | down |
| dystroglycan 1 | CfaAffx.17467.1.S1_at | up |

**Table. 11 Genes associated with Lipid metabolism**

| | | **Direction of expression** |
|---|---|---|
| **Annotation** | **Probe** | **H v. P** |
| Toll-like receptor 2 | CfaAffx.13248.1.S1_s_at | Down |
| cytochrome P450, family 1, subfamily B, polypeptide 1 | CfaAffx.10229.1.S1_at | Down |
| colony stimulating factor 1 receptor | CfaAffx.27899.1.S1_at | Down |
| phospholipase C, beta 1 | Cfa.10853.1.A1_at | Down |
| fibronectin 1 | Cfa.3707.3.S1_s_at | Down |
| neuregulin 1 | CfaAffx.10523.1.S1_s_at | Down |
| macrophage receptor with collagenous structure | Cfa.15713.1.A1_at | Down |
| CD1a molecule | Cfa.18390.1.S1_s_at | Down |

**Table. 12 Genes associated with Cardiovascular development**

| | | **Direction of expression** |
|---|---|---|
| **Annotation** | **Probe** | **H v. P** |
| bactericidal/permeability-increasing protein | | |
| | CfaAffx.14056.1.S1_s_at | Down |
| fibronectin 1 | | |
| | Cfa.3707.3.S1_s_at | Down |
| neuregulin 1 | | |
| | CfaAffx.10523.1.S1_s_at | Down |
| xanthine dehydrogenase | | |
| | CfaAffx.9452.1.S1_s_at | Down |
| annexin A6 | | |
| | CfaAffx.27471.1.S1_s_at | Down |
| colony stimulating factor 1 receptor | | |
| | CfaAffx.27899.1.S1_at | Down |
| alanyl (membrane) aminopeptidase | | |
| | Cfa.3774.1.A1_s_at | Down |

**Table. 13 Genes associated with Skeletal/Muscular disorders and contraction/function**

| | | **Direction of expression** |
|---|---|---|
| **Annotation** | **Probe** | **H v. P** |
| **Skeletal/Muscular disorders** | | |
| Toll-like receptor 2 | CfaAffx.13248.1.S1_s_at | down |
| colony stimulating factor 1 receptor | CfaAffx.27899.1.S1_at | down |
| fibronectin 1 | Cfa.3707.3.S1_s_at | down |
| neuregulin 1 | CfaAffx.10523.1.S1_s_at | down |

| **Muscle contraction/function** | | |
|---|---|---|
| exostoses | Cfa.11001.1.A1_at | up |
| microfibrillar associated protein 5 | CfaAffx.21449.1.S1_s_at | up |
| myosin light polypeptide kinase | Cfa.13192.1.S1_at | up |
| dystroglycan 1 | CfaAffx.17467.1.S1_at | up |
| Moloney leukemia virus 10-like 1 | CfaAffx.1982.1.S1_s_at | up |

**Table. 14 Genes associated with Cellular Compromise**

| | | **Direction of expression** |
|---|---|---|
| **Annotation** | **Probe** | **Hv. P** |
| neuroregulin 1 | CfaAffx.10523.1.S1_s_at | down |
| fibronectin 1 | Cfa.3707.3.S1_s_at | down |
| macrophage receptor with collagenous structure | Cfa.15713.1.A1_at | down |
| xanthine dehydrogenase | CfaAffx.9452.1.S1_s_at | down |
| bactericidal permeability increasing protein | CfaAffx.14056.1.S1_s_at | down |
| phospholipase C, beta 1 | Cfa.10853.1.A1_at | down |
| hemoglobin epsilon 1 | CfaAffx.10240.1.S1_s_at | up |

**Table. 15 Genes associated with various functions**

| | | **Direction of expression** |
|---|---|---|
| **Annotation** | **Probe** | **H v. P** |
| CCR4-NOT transcription complex, subunit 10 | Cfa.2403.1.A1_at | up |
| ubiquitin carboxy-terminal hydrolase L5 | Cfa.20277.1.S1_at | up |
| SH3-domain GBR2-like 3 | Cfa.10644.1.A1_at | up |
| DEAH (Asp-Glu-Ala-His) box polypeptide 8 | CfaAffx.22452.1.S1_s_at | up |
| Signal-induced proliferation-associated 1 like 2 | Cfa.14345.1.A1_at | up |
| Cathepsin E | CfaAffx.15966.1.S1_at | up |
| ephrin-B3 | Cfa.15231.1.A1_at | up |
| Fc fragment of IgG, receptor, transporter, alpha | Cfa.17806.2.S1_s_at | up |
| Chromosome 20 open reading frame 172 | Cfa.9662.1.A1_at | up |
| Inositol(myo)-1(or 4) monophosphatase 2 | Cfa.13029.1.A1_at | up |
| Ferrochelatase (protoporphyria) | Cfa.365.3.A1_x_at | up |
| G-protein coupled receptor 158 | Cfa.9162.1.A1_at | up |
| dual specificity phosphatase 19 | CfaAffx.22249.1.S1_at | up |
| Ring finger protein 152 | Cfa.15414.1.A1_at | up |
| Chromosome 12 open reading frame 49 | Cfa.11298.1.S1_at | up |
| hemoglobin, epsilon 1 | CfaAffx.10240.1.S1_s_at | up |
| Crystallin lambda 1 | Cfa.4354.1.S1_a_at | up |
| Sorting nexin 6 | Cfa.10261.1.S1_at | up |
| microfibrillar associated protein 5 | CfaAffx.21449.1.S1_s_at | up |
| coiled-coil domain containing 46 | CfaAffx.17557.1.S1_s_at | up |
| Nuclear factor 1/B | Cfa.4487.1.S1_at | up |
| carboxypeptidase A3 | CfaAffx.13076.1.S1_at | up |
| chromosome 18, clone RP11-396D4 | CfaAffx.26602.1.S1_at | up |
| coxsackie and adenovirus receptor protein | CfaAffx.13148.1.S1_at | up |
| WW domain containing oxidoreductase, transcript variant 4 | Cfa.9201.1.A1_at | up |
| EH domain binding protein 1-like 1 | Cfa.17641.1.S1_s_at | Down |
| RP4-760G15 on chromosome 11p13 | Cfa.17214.1.S1_at | Down |
| phospholipase C beta 1 (phosphoinositide-specific) | Cfa.10853.1.A1_at | Down |
| cathepsin L | CfaAffx.2868.1.S1_at | Down |
| Transcripdon elongation factor A, 1 | Cfa.12580.1.S1_s_at | Down |
| glypican 6 | Cfa.9523.1.A1_at | Down |
| Hypothetical protein FLJ21062 | Cfa.1933.2.A1_at | Down |
| G-protein coupled receptor 101 | CfaAffx.12632.1.S1_at | Down |
| Leucine-rich repeat kinase 2 | CfaAffx.15613.1.S1_s_at | Down |
| Six transmembrane epithelial anitgen of the prostate 2 | Cfa.9430.1.A1_at | Down |
| KIAA1074 protein | C1fa.12143.1.A1_at | down |
| LOC284395 | Cfa.4802.1.S1_at | down |
| elastin microfibril interfacer 2 | CfaAffx.28185.1.S1_at | down |
| Sulfatase 2 | CfaAffx.17034.1.S1_s_at | down |
| IBR domain containing 2 | Cfa.1882.1.A1_at | down |
| Leucyl/cystinyl aminopeptidase | CfaAffx.12483.1.S1_s_at | down |
| acyltransferase like 1 | CfaAffx.14833.1.S1_at | down |
| NAD kinase | CfaAffx.29324.1.S1_at | down |
| Immunoglobulin heavy constant alpha 2 | Cfa.4556.3.A1_x_at | down |
| transcription factor EC (TFEC), transcript variant 2 | Cfa.1175.1.A1_s_at | down |
| Acyl-CoA synthetase short-chain family member 1 | CfaAffx.12483.1.S1_at | down |
| Syntaxin binding protein 6 | Cfa.9054.1.A1_at | down |
| Opioid growth factor receptor-like 1 | Cfa.583.1.S1_at | down |
| None | Cfa.10106.1.A1_at | down |
| None | Cfa.4556.3.A1_s_at | down |
| ELK/RAB6-interacting/CAST family member 2 | Cfa.21214.1.S1_at | down |
| adipsin/complement factor D precursor | Cfa.21381.1.S1_s_at | down |
| Hypothetical protein FLJ22662 | CfaAffx.20271.1.S1_at | down |
| solute carrier family 2 (facilitated glucose transporter), member 9 | Cfa.7132.1.A1_at | down |
| Homogentisate 1,2 dioxygenase | CfaAffx.9076.1.S1_s_at | down |
| serpin peptidase inhibitor, clade B (ovalbumin), member 10 | CfaAffx.1043.1.S1_s_at | down |
| None | Cfa.13203.1.S1_at | down |
| Fibrinogen-like 2 | CfaAffx.7369.1.S1_s_at | down |
| None | Cfa.12500.1.A1_at | down |
| None | Cfa.4556.2.S1_s_at | down |
| None | Cfa.4555.1.S1_s_at | down |
| None | Cfa.280.1.S1_at | down |
| Lymphocyte antigen 6 | CfaAffx.1839.1.S1_at | down |
| Neuronal PAS domain protein 3 | Cfa.12905.1.A1_a_at | down |

Gene expression profiles from puppies fed Composition H or P from weaning to one year of age are obtained and compared. Microarray data indicate that, at a minimum of 1.5 fold change, 99 genes are differentially expressed in the two study groups.

Genes associated with immune activation are down-regulated in puppies fed Composition H when compared to puppies fed Composition P, which suggests improved immune system function (Table 9).

Genes associated with lipid metabolism are down-regulated in puppies fed Composition H when compared to puppies fed Composition P, which suggests reduced lipid processing (Table 11).

Genes associated with cardiovascular development are also down-regulated in puppies fed Composition H when compared to puppies fed Composition P, which suggests enhanced cardiovascular health (Table 12).

Genes associated with skeletal muscular disorders are down-regulated and genes associated with muscle contraction/function are up-regulated in puppies fed Composition H when compared to puppies fed Composition P, which suggests reduced skeletal disorder risk and improved muscle contraction (Table 13).

Genes associated with cellular compromise are down-regulated in puppies fed Composition H when compared to puppies fed Composition P, which suggests reduced cellular damage (Table 14).

Genes known to have an association with cancer are down-regulated in puppies fed Composition H when compared to puppies fed Composition P, which may suggest reduced cancer susceptibility. (Table 10).

As discussed above, genetic data such as these indicate that the nutritional benefits of the compositions of the present invention include the beneficial modification of gene expression in the animal such that there is an overall enhancement in the development of the animal. In addition, the beneficial modification of gene expression may also result in a decrease in the incidence of disease in the animal due to an inhibition in expression of disease related genes and/or an increase in the expression of genes which play a role in disease prevention.

## Claims

1. A pet food composition comprising:
5 to 70% protein,
0.5 to 1.6% methionine,
50 to 200 ppm manganese,
0.1 to 0.5% DHA,
0.1 to 0.7% EPA,
1200 to 7500 ppm choline,
1000 to 2000 ppm taurine,
2.5 to 6 % linoleic acid,
1 to 3% total n-3 fatty acids,
50 to 1200 IU/ kg vitamin E,
50 to 500 ppm vitamin C,
50 to 500 ppm carnitine, and
2.5 to 7g lysine/ 1000 kcal,
wherein the composition is for use in preventing or treating arthritis or bone and joint inflammation, or in preventing or treating cancer in a canine,
wherein the use comprises regulating gene expression in the canine,
wherein the use comprises feeding a dam prior to and during pregnancy, wherein the canine is born of the dam fed the composition prior to and during pregnancy,
and wherein the use further comprises feeding the canine the composition for at least one year post-partum.

2. The composition for use according to claim 1 comprising:
0 to 90% by weight of carbohydrates;
20% to 60% by weight of protein;
2% to 50% by weight of fat;
0.1% to 20%, by weight of total dietary fiber; and
0 to 15% by weight of vitamins, minerals, and other nutrients.

3. The composition for use according to claim 1 or 2 comprising 0.1% to 0.4% DHA, and/or comprising 100 ppm to 500 ppm carnitine, and/or comprising 2.5 g/ 1000 kcal to 7 g/ 1000 kcal lysine, and/or comprising 0.1 % to 0.6 % EPA, and/or comprising 50 ppm to 150 ppm manganese, and/or comprising 0.8% to 1.6% methionine.

4. The composition for use according to any of claims 1 to 3 wherein the use comprises feeding the dam the composition for a majority of the pregnancy duration.

## Patentansprüche

1. Tierfutterzusammensetzung, die Folgendes umfasst:
5 bis 70 % Protein,
0,5 bis 1,6 % Methionin,
50 bis 200 ppm Mangan,
0,1 bis 0,5 % DHA,
0,1 bis 0,7 % EPA,
1200 bis 7500 ppm Cholin,
1000 bis 2000 ppm Taurin,
2,5 bis 6 % Linolsäure,
1 bis 3 % Gesamt-n-3-Fettsäuren,
50 bis 1200 IE/kg Vitamin E,
50 bis 500 ppm Vitamin C,
50 bis 500 ppm Carnitin und
2,5 bis 7 g Lysin/1000 kcal,
wobei die Zusammensetzung zur Verwendung beim Verhindern oder Behandeln von Arthritis oder Knochen- und Gelenkentzündung oder beim Verhindern oder Behandeln von Krebs bei einem Hund ist,
wobei die Verwendung das Regulieren der Genexpression in dem Hund umfasst,
wobei die Verwendung das Füttern eines Muttertiers vor und während einer Trächtigkeit umfasst,
wobei der Hund von dem Muttertier geboren wird, das mit der Zusammensetzung vor und während der Trächtigkeit gefüttert wurde,
und wobei die Verwendung weiterhin das Füttern des Hunds mit der Zusammensetzung für mindestens ein Jahr nach der Geburt umfasst.

2. Zusammensetzung zur Verwendung nach Anspruch 1, die Folgendes umfasst:
0 bis 90 Gew.-% Kohlenhydrate;
20 Gew.-% bis 60 Gew.-% Protein;
2 Gew.-% bis 50 Gew.-% Fett;
0,1 Gew.-% bis 20 Gew.-% Gesamtballaststoffe und
0 bis 15 Gew.-% Vitamine, Mineralien und andere Nährstoffe.

3. Zusammensetzung zur Verwendung nach Anspruch 1 oder 2, die 0,1 % bis 0,4 % DHA umfasst und/oder 100 ppm bis 500 ppm Carnitin umfasst und/oder 2,5 g/1000 kcal bis 7 g/1000 kcal Lysin umfasst und/oder 0,1 % bis 0,6 % EPA umfasst und/oder 50 ppm bis 150 ppm Mangan umfasst und/oder 0,8 % bis 1,6 % Methionin umfasst.

4. Zusammensetzung zur Verwendung nach einem der Ansprüche 1 bis 3, wobei die Verwendung das Füttern des Muttertiers mit der Zusammensetzung für einen Großteil der Trächtigkeitsdauer umfasst.

## Revendications

1. Une composition alimentaire pour animaux de compagnie comprenant :
de 5 à 70% de protéines,
de 0,5 à 1,6% de méthionine,
50 à 200 ppm de manganèse,
de 0,1 à 0,5% de DHA,
de 0,1 à 0,7% d'EPA,
de 1200 à 7500 ppm de choline,
de 1000 à 2000 ppm de taurine,
de 2,5 à 6% d'acide linoléique,
de 1 à 3% du total des n-3 d'acides gras,
de 50 à 1200 IU/ kg de vitamine E,
de 50 à 500 ppm de vitamine C,
de 50 à 500 ppm de carnitine, et
de 2,5 à 7 g de lysine / 1000 kcal,
dans laquelle la composition doit être utilisée dans la prévention ou le traitement de l'arthrite ou des inflammations osseuses et articulaires, ou dans la prévention ou le traitement du cancer chez le chien,
dans laquelle l'utilisation comprend la régulation de l'expression génique chez le chien,
dans laquelle l'utilisation comprend l'alimentation de la mère avant et pendant la grossesse, dans laquelle le chien nait d'une mère nourrie avec la composition avant et pendant la grossesse,
et dans laquelle l'utilisation comprend en outre l'alimentation du chien avec la composition pendant au moins un an post partum.

2. La composition pour utilisation selon la revendication 1 comprenant :
de 0 à 90% en poids de glucides ;
de 20% à 60% en poids de protéines ;
de 2% à 50% en poids de matières grasses ;
de 0,1% à 20%, en poids des fibres alimentaires totales ; et
de 0 à 15% en poids de vitamines, minéraux et autres nutriments.

3. La composition pour une utilisation selon la revendication 1 ou 2 comprenant de 0,1% à 0,4% de DHA, et/ou comprenant de 100 ppm à 500 ppm de carnitine, et/ou comprenant de 2,5 g / 1000 kcal à 7 g / 1000 kcal de lysine, et/ou comprenant de 0,1% à 0,6% d'EPA et/ou comprenant de 50 ppm à 150 ppm de manganèse, et/ou comprenant de 0,8% à 1,6% de méthionine.

4. La composition pour une utilisation selon l'une quelconque des revendications 1 à 3 dans laquelle l'utilisation comprend l'alimentation de la mère avec la composition pendant la plus grande partie de la durée de la grossesse.
